# EUROPEAN PATENT SPECIFICATION

(11) **EP 2 398 528 B1**
(45) Date of publication and mention of the grant of the patent: **12.08.2015**
(21) Application number: 10705072.6
(22) Date of filing: 19.02.2010
(51) Int. Cl.: A61M 1/28

(54) **BULK DELIVERY PERITONEAL DIALYSIS SYSTEM AND METHOD**
BULK-PERITONEALDIALYSESYSTEM UND VERFAHREN
SYSTÈME ET PROCÉDÉ DE DIALYSE PÉRITONÉALE À ADMINISTRATION EN VRAC

(30) Priority: 20.02.2009 US 390002
(43) Date of publication of application: 28.12.2011
(73) Proprietor: Baxter International Inc., Deerfield, IL 60015 (US); Baxter Healthcare SA, 8152 Glattpark (Opfikon) (CH)
(72) Inventor: LO, Ying-Cheng, Green Oaks Illinois 60048 (US); VISHNOI, Rohit, Deerfield Illinois 60015 (US)
(74) Representative: Potter Clarkson LLP
(86) International application number: PCT/US2010/024742
(87) International publication number: WO 2010/096657

(56) References cited:
- EP-A1- 1 389 473
- WO-A1-83/01573
- JP-A- 9 173 447
- US-A- 3 545 438
- US-A- 5 722 947
- US-A1- 2003 225 066
- US-A1- 2004 097 862

## Description

### BACKGROUND

The present disclosure relates to medical fluid delivery and in particular to peritoneal dialysis fluid delivery.

Due to disease, insult or other causes, a person's renal system can fail. In renal failure of any cause, there are several physiological derangements. The balance of water, minerals and the excretion of daily metabolic load is no longer possible in renal failure. During renal failure, toxic end products of nitrogen metabolism (urea, creatinine, uric acid, and others) can accumulate in blood and tissues.

Kidney failure and reduced kidney function have been treated with dialysis. Dialysis removes waste, toxins and excess water from the body that would otherwise have been removed by normal functioning kidneys. Dialysis treatment for replacement of kidney functions is critical to many people because the treatment is life saving. One who has failed kidneys could not continue to live without replacing at least the filtration functions of the kidneys.

One type of dialysis is peritoneal dialysis. Peritoneal dialysis uses a dialysis solution or "dialysate", which is infused into a patient's peritoneal cavity through a catheter implanted in the cavity. The dialysate contacts the patient's peritoneal membrane in the peritoneal cavity. Waste, toxins and excess water pass from the patient's bloodstream through the peritoneal membrane and into the dialysate. The transfer of waste, toxins, and water from the bloodstream into the dialysate occurs due to diffusion and osmosis, i.e., an osmotic gradient occurs across the membrane. The spent dialysate drains from the patient's peritoneal cavity and removes the waste, toxins and excess water from the patient. This cycle is repeated.

There are various types of peritoneal dialysis therapies, including continuous ambulatory peritoneal dialysis ("CAPD") and automated peritoneal dialysis ("APD"). CAPD is a manual dialysis treatment, in which the patient connects an implanted catheter to a drain and allows a spent dialysate fluid to drain from the patient's peritoneal cavity. The patient then connects the catheter to a bag of fresh dialysate and manually infuses fresh dialysate through the catheter and into the patient's peritoneal cavity. The patient disconnects the catheter from the fresh dialysate bag and allows the dialysate to dwell within the cavity to transfer waste, toxins and excess water from the patient's bloodstream to the dialysate solution. After a dwell period, the patient repeats the manual dialysis procedure.

In CAPD the patient performs several drain, fill, and dwell cycles during the day, for example, about four times per day. Each treatment cycle typically takes about four hours. APD is similar to CAPD in that the dialysis treatment includes a drain, fill, and dwell cycle. APD machines, however, perform three to four cycles of peritoneal dialysis treatment automatically, typically overnight while the patient sleeps. Like CAPD, APD machines connect fluidly to an implanted catheter, to one or more sources or bags of fresh dialysate and to a fluid drain.

The APD machines pump fresh dialysate from the dialysate source, through the catheter, into the patient's peritoneal cavity and allow the dialysate to dwell within the cavity so that the transfer of waste, toxins and excess water from the patient's bloodstream to the dialysate solution can take place. The APD machines then pump spent dialysate from the peritoneal cavity, though the catheter, to the drain. APD machines are typically computer controlled so that the dialysis treatment occurs automatically when the patient is connected to the dialysis machine, for example, when the patient sleeps. That is, the APD systems automatically and sequentially pump fluid into the peritoneal cavity, allow for a dwell, pump fluid out of the peritoneal cavity and repeat the procedure. As with the manual process, several drain, fill, and dwell cycles will occur during APD. A "last fill" is typically used at the end of APD, which remains in the peritoneal cavity of the patient when the patient disconnects from the dialysis machine for the day.

While, APD frees the patient from having to manually performing the drain, dwell, and fill steps, a need still exists for CAPD. Some patients prefer the control that CAPD offers. Since the patient is awake during CAPD, the patient can adjust himself/herself during drain to produce more complete drains. Further, many patients who perform APD also perform a midday exchange using a CAPD technique. A continued need therefore exists for simple (for example, for patients performing exchanges at work) and effective CAPD systems.

US 2003/0225066 is concerned with a peritoneal dialysis solution with taurolidine. The document discloses a fluid set that comprises a plurality of dialysis fluid bags or reservoirs and an arrangement of fluid lines that connect the fluid bags to a central junction.

JP 09, 173447 discloses a dosing apparatus comprising a plurality of containers each holding a separate component for a medicine and a plurality of tubes that connect the containers to a central Y-shaped junction.

### SUMMARY

According to the present invention there is provided a peritoneal dialysis set according to claim 1 and a peritoneal dialysis system according to claim 12.

The present disclosure sets forth a peritoneal dialysis set and corresponding system and method for performing peritoneal dialysis. As seen below, the set requires relatively little apparatus, and the method involves relatively simple clamping and unclamping steps in combination with the movement of a small, e.g., two liter, supply bag.

The set and treatment are intended for patients who prefer continuous ambulatory peritoneal dialysis ("CAPD") or who need to perform one or more manual midday exchanges in combination with nightly automated peritoneal dialysis ("APD").

The set includes a larger supply bag, e.g., six liters, that is connected fluidly to a smaller, e.g., two liter, supply bag via a supply tube. The larger and smaller supply bags are both initially full and hold the patient's prescribed total treatment volume collectively in one embodiment (plus a small amount used for flushing). The smaller supply bag is provided with two ports, one port for connecting to the larger supply bag via a supply line and a second port for connecting to a three-way connector or junction, such as a Y-junction or T-junction via a fill tube.

The three-way connector or junction is also coupled to a drain line and has a patient connector for patient connection. The drain line runs to a house drain or drain container, such as a reusable drain container. The patient connector connects to a patient's transfer set, which leads to a catheter implanted inside the patient's peritoneum. Both the patient connector and the patient's transfer set are sealed originally with a cap that is removed for therapy.

In one arrangement, the patient performs treatment using just two manually operated clamps, such as external/removable clamps, or fixed clamps such as Roberts™ clamps. To perform a first exchange, the patient uses a first clamp to prevent fresh dialysate from flowing from the larger supply container to the smaller supply container and a second clamp to prevent fresh dialysate from flowing from the smaller supply container to the three-way connector or junction. The patient also secures the drain line to the three-way connector or junction. For example, the patient can remove a cap from the drain line and secure the drain line to a drain container, e.g., reusable container, or run the drain line to a house drain, such as a toilet or bathtub. All of the above can be done before the patient connects the three-way connector or junction to the patient's transfer set.

To accomplish the drain phase, the patient removes disposable caps from both the patient's transfer set and the patient connector of the three-way connector or junction and connects the patient connector to the patient's set (which is closed). The patient stands or sits above the house or reusable drain and opens the transfer set so that the spent dialysis fluid residing already in the patient's peritoneum from the last therapy is allowed to gravity flow to drain. The patient can maneuver himself/herself to allow the spent fluid to drain from the patient effectively. The supply and fill tubes remain clamped, and while the patient is waiting for the drain phase to be completed, the patient can hang the smaller supply container above the larger supply container to ready for the next step.

To accomplish a flush step, the patient closes the transfer set valve and opens the manual clamp on the fill tube, allowing fresh dialysate to gravity flow from the smaller supply container to the house or reusable drain. The clamp on the supply tube between the larger and smaller supply containers remains clamped. The patient allows fresh dialysate to gravity flow through the fill tube, though the three-way connector or junction, and through the drain line to drain, flushing the fill tube, junction and drain tube. The patient allows the flush to continue for a specified period of time or until visual inspection indicates that the flush is finished.

To accomplish a fill phase, the patient moves the opened clamp on the fill tube to the drain tube and clamps the drain tube. The patient opens the transfer set valve, so that fresh fluid can gravity flow from the smaller supply container, through the fill tube, though the junction, the patient's transfer set and into the patient's peritoneal cavity. The patient allows the smaller supply container to empty the remainder of its contents into the peritoneal cavity or until the patient feels full.

To accomplish a dwell phase, the patient opens and moves the closed clamp on the supply tube to the fill tube and clamps the fill tube, such that the fill and drain tubes are both clamped. The patient then disconnects the patient's transfer set from the patient connector of the junction and recaps both the transfer set and the patient connector using fresh caps. In one method, the smaller supply container is left held elevationally above the larger supply container during dwell, such that the smaller container is not refilled at this time. In the one method, the larger supply container is left in place, e.g., on a table so as to be at approximately a same height as the patient's access, during the entire therapy. The patient can then leave and perform whatever task the patient wishes during the dwell period.

For a second exchange, the patient moves the smaller supply container from the elevated fill position to a low elevational refill position, below the larger supply container, which is laid on a table above the smaller supply container. If the supply tube is clamped, the clamp is opened to allow fresh dialysate to gravity flow from the larger supply container to refill the smaller supply container. All above steps can be done during the dwell phase after disconnecting the transfer set from the patient connectors. That is, the patient does not have to wait for the second exchange to begin before preparing for the next exchange.

During the next exchange, The patient moves the second clamp from the drain tube to the fill tube to ready the set for the next drain. The patient then removes the caps from the transfer set and patient connector and connects the patient transfer set (which is closed) to the connector of the junction of the dialysis set. The patient moves the refilled smaller supply container to the elevated position above the larger supply container, while ensuring that the supply tube is clamped to prevent dialysate from flowing back from the smaller supply container to the larger supply container. A second clamp is used to prevent dialysate from flowing from the supply bag to the patient because the patient needs to drain first before beginning the next fill. The set is now ready for a second drain, which is initiated when the patient opens his/her transfer set.

When all exchanges, e.g., four exchanges, have been completed, the patient recaps the transfer set with a new cap, removes and saves the manual clamps. The patient removes the drain line from the drain and empties the reusable container if one is used and discards the peritoneal dialysis set. The clamps and drain can be reused until they need too be replaced.

It is accordingly an advantage of the present disclosure to provide a continuous ambulatory peritoneal dialysis ("CAPD") set that requires little apparatus.

It is another advantage of the present disclosure to provide a continuous ambulatory peritoneal dialysis ("CAPD") set that is easy to use.

Additional features and advantages are described herein, and will be apparent from the following Detailed Description and the figures.

### BRIEF DESCRIPTION OF THE FIGURES

Fig. 1 illustrates one embodiment of a peritoneal dialysis set of the present disclosure.
Fig. 2 illustrates a peritoneal dialysis system using the set of the present disclosure, which is about to be connected to a patient and a drain container to begin a first exchange.
Fig. 3 is a therapy flow diagram illustrating one embodiment for using the peritoneal dialysis set of the present disclosure to perform a multiple exchange therapy.
Fig. 4 illustrates one embodiment for performing a drain phase using the peritoneal dialysis set of the present disclosure.
Fig. 5 illustrates one embodiment for performing a flushing step using the peritoneal dialysis set of the present disclosure.
Fig. 6 illustrates one embodiment for performing a filling phase using the peritoneal dialysis set of the present disclosure.
Fig. 7 illustrates one embodiment for performing a dwell phase using the peritoneal dialysis set of the present disclosure.
Figs. 8 and 9 illustrate one embodiment for transitioning from the first exchange to a second exchange using the peritoneal dialysis set of the present disclosure.
Fig. 10 illustrates one embodiment for ending a therapy using the peritoneal dialysis set of the present disclosure.

### DETAILED DESCRIPTION

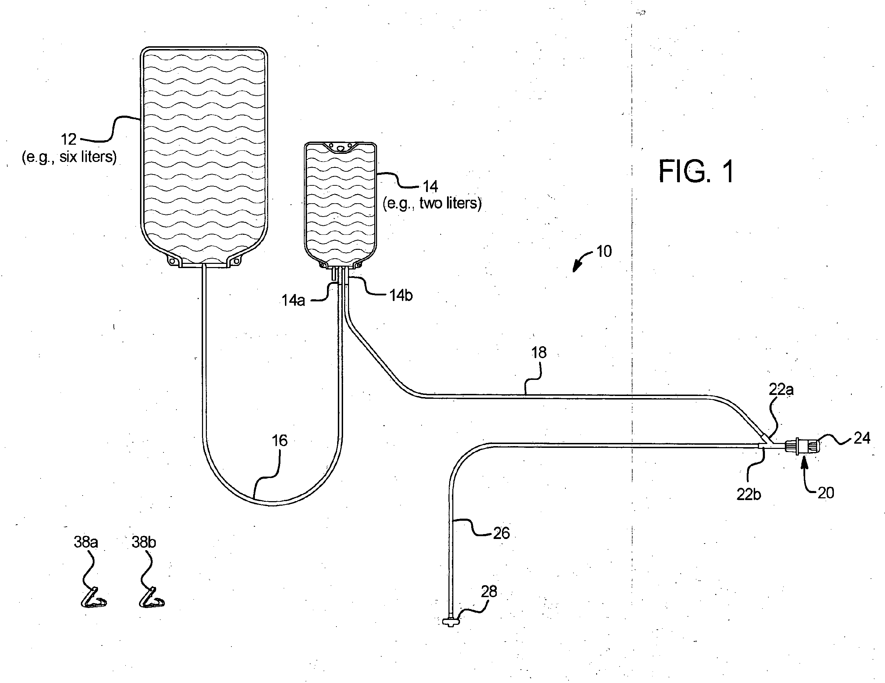

Referring now to the drawings and in particular to Fig. 1, one embodiment of a peritoneal dialysis set is illustrated by set 10. Set 10 and its corresponding treatment are for patients who prefer continuous ambulatory peritoneal dialysis ("CAPD") or who need to perform one or more manual midday exchange in combination with nightly automated peritoneal dialysis ("APD").

Set 10 includes a larger supply bag 12, e.g., six liters, that is connected fluidly to the smaller, e.g., two liter, supply bag 14 via a supply tube 16. The larger and smaller supply bags 12 and 14 are both initially full and hold the patient's prescribed total treatment volume (e.g., eight liters) collectively in one embodiment (plus an extra amount for flushing). Smaller supply bag 14 is provided with two ports 14a and 14b, one port 14a for connecting to larger supply bag 12 via supply line 16 and a second port 14b for connecting to a fill line 18 leading to a three-way connector or junction 20, such as a Y-junction or T-junction.

In an alternative embodiment, port 14a is fitted with a frangible. The frangible is unbroken for the first fill, which comes from smaller supply bag 14 and carries a first type of dialysate. After the first fill, the frangible is broken, so that a second dialysate type, different from the first can be delivered from larger supply bag 12 to smaller supply bag 14, and then delivered to the patient (process for delivering from container 12 to container 14 and then to the patient is discussed in detail below). The frangible accordingly allows different types of fluids to be delivered to the patient. For example, the first fill from smaller supply bag 14 could hold a more expensive type of dialysate. The assignee of the present disclosure makes a dialysate called Extraneal™, which is polyglucose-based as opposed to a dextrose-based dialysate (e.g.., Dianeal™) and can thereby pull ultrafiltrate ("UF") from the patient for a longer period of time, e.g., eight hours, than can the standard dextrose-based dialysate (which may lose UF removal capability after four hours). The Extraneal™ dialysate is initially filled into the patient just prior to a long day or overnight dwell. Afterwards, the Dianeal™ dialysate is filled from the larger supply bag 12 for shorter dwells.

In certain countries, a more expensive dialysate, such as Extraneal™ dialysate, is too expensive. It is expressly contemplated herein to offset the additional expense of the dialysate by reducing the disposable cost via set 10. Set 10 therefore yields an overall better therapy for the patient in such countries.

Three-way connector or junction 20 includes a first port 22a that is connected to fill tube 18. A second port 22b is connected to a drain tube 26. Junction 20 includes a third port or patient connector 24, which is configured to mate in a releasable and fluid tight manner (e.g., threaded with o-ring seal) with a patient's transfer set (shown below as set 40), which leads to a catheter implanted inside the patient's peritoneum.

Drain tube 26 runs to a house drain or drain container, such as a reusable drain container shown below as container 30. In the illustrated embodiment, drain tube 26 includes a removable handle and plug 28. Set 10 also includes reusable manual clamps 38a and 38b, such as reusable line clamps provided by Baxter International Inc., the use of which is described below. Reusable manual clamps 38a and 38b can be used over and over again until they need to be replaced.

In one implementation, supply bags 12 and 14 are made of medical grade polymers, e.g., PVC or polyolefin-based non-PVC material. Tubes 16, 18 and 26 are made of PVC or polyolefin-based non-PVC material. Junction 20 including connector 24 can be made of Hytrel, PVC or polycarbonate. Supply tube 16 can be about 2.5 feet in length. Fill and drain tubes 18 and 26 can be about 0.91 metres (three feet) in length.

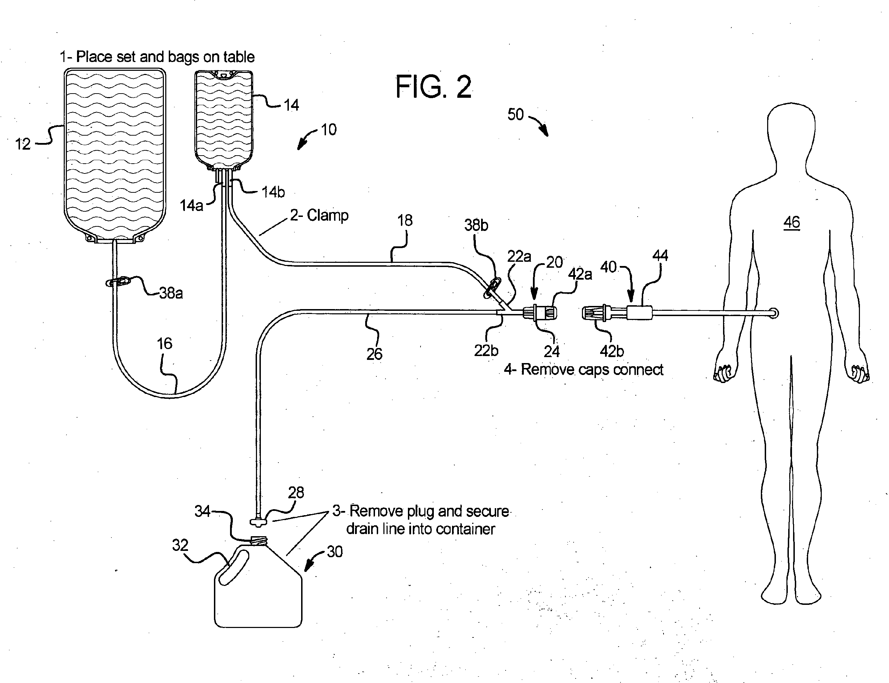

Referring now to Fig. 2, a peritoneal dialysis system 50 using set 10 is illustrated. System 50 includes drain container 30 and transfer set 40. Drain container 30 includes a handle 32 for moving the container. Container 30 also includes a threaded opening 34 for receiving a cap (not illustrated) when the container is not in use. The opening of container 30 in one embodiment includes a clip or other suitable apparatus (not illustrated) for securing drain tubing 26 to the container.

Patient connector 24 of junction 20 is capped via a removable cap 42a. Patient transfer set 40 is likewise sealed originally via a cap 42b that is removed for therapy. Patient transfer set 40 also includes a twistable valve 44 that allows patient 46 to open and occlude flow through transfer set 40.

### Using Individual Manual Clamps

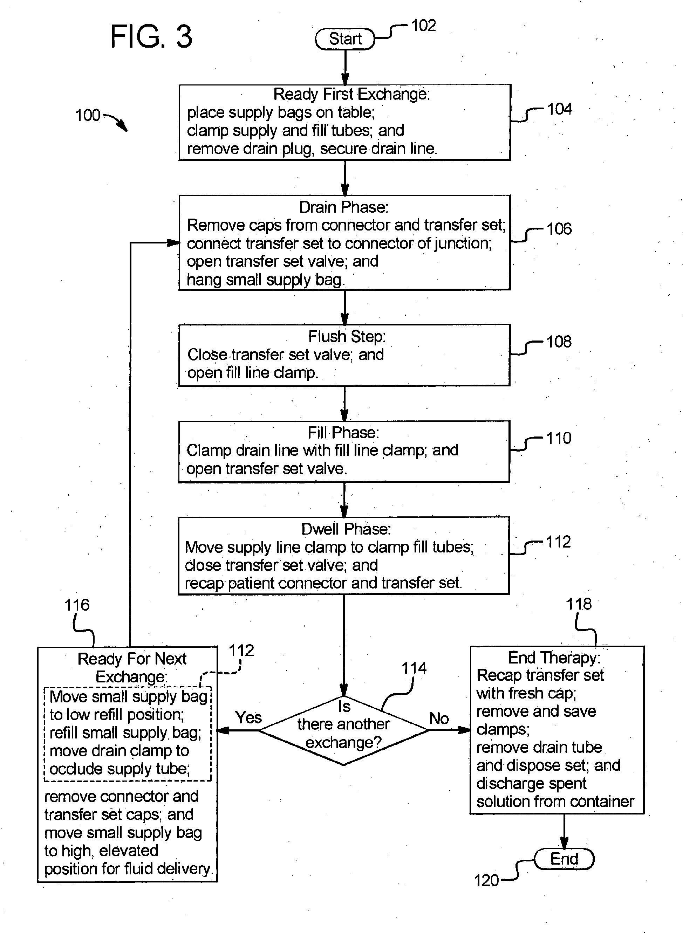

Referring additionally to the therapy method 100 of Fig. 3, in one embodiment, patient 46 can perform treatment using just the two manually operated external clamps 38a and 38b. Method 100 begins at oval 102, and at block 104, patient 46 readies the first exchange. To ready the first exchange, patient 46 places both supply containers 12 and 14 on a table or other piece of furniture such that the bags are elevated above drain container 30. The patient clamps first clamp 38a to prevent fresh dialysate from flowing from larger supply container 12 to smaller supply container 14, and clamps second clamp 38b to prevent fresh dialysate from flowing from smaller supply container 14 to the three-way connector or junction 20.

At block 104, patient 46 also secures drain line 26 to port 22b of three-way connector or junction 20. For example, the patient can remove plug cap 28 from drain line 26 and secure the drain line to drain container 30 (or secure the drain line to a house drain, such as a toilet or bathtub). As shown in Fig. 2, the actions of block 104 are all performed in one embodiment before patient 46 connects the three-way connector or junction 20 to the patient's transfer set 40.

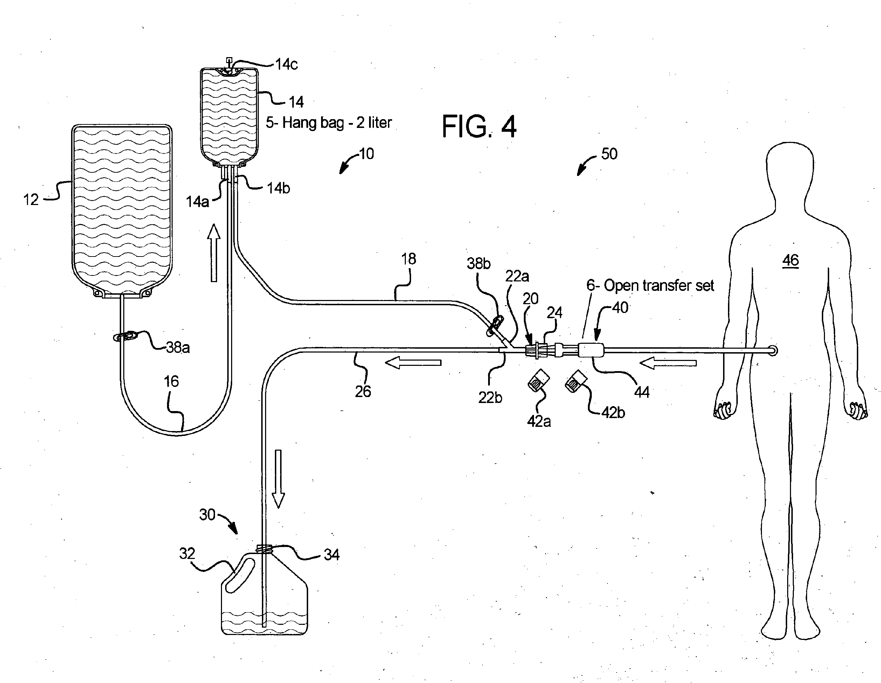

Referring now to Fig. 4 and block 106 of Fig. 3, to accomplish the drain phase, the patient removes disposable caps 42a and 42b from both the patient's transfer set 40 and the patient connector 24 of the three-way connector or junction 20, respectively, and connects the patient connector 24 to the patient's set 40 (which is closed). Patient 46 stands or sits above the house or reusable drain 30 and opens twistable valve 44 of transfer set 40, so that the spent dialysis fluid residing already in the peritoneum of patient 46 from the last therapy is allowed to gravity flow to drain 30. Patient 46 can maneuver himself/herself to help the spent fluid to drain completely from the patient. As seen in Fig. 4, the supply and fill tubes 16 and 18 remain clamped. Also, while the patient is waiting for the drain phase to be completed, patient 46 can hang smaller supply container 14 above the larger supply container 12 to ready for the next step. For example, patient 46 can loop a tab 14c of smaller supply container 14 forming a hanging hole or aperture onto an elevated hook or pole.

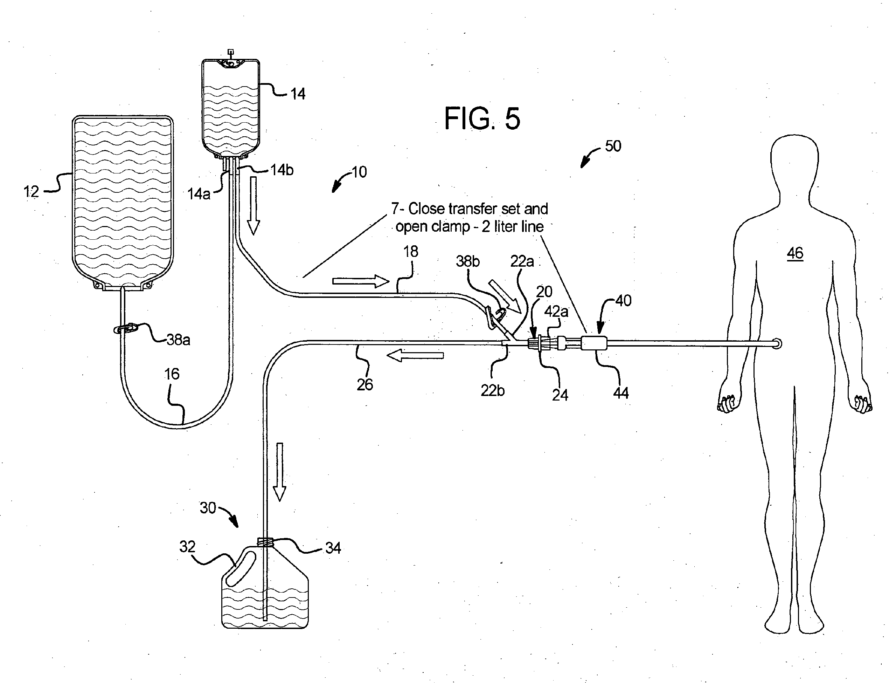

Referring now to Fig. 5 and block 108 of Fig. 3, to accomplish a flush step, the patient closes transfer set valve 44 and then opens manual clamp 38b on the fill tube 18, allowing fresh dialysate to gravity flow from smaller supply container 14 to the house or reusable drain 30. Clamp 38a (the only one needed during flush) on supply tube 16 between the larger and smaller supply containers 12 and 14 remains clamped. The patient allows fresh dialysate to gravity flow through the fill tube 18, though the three-way connector or junction 20, and through drain line 26 to drain 30, flushing the fill tube 18, junction 20 and drain tube 26. Patient 46 allows the flush phase to continue for a specified period of time or until visual inspection indicates that the flush is finished.

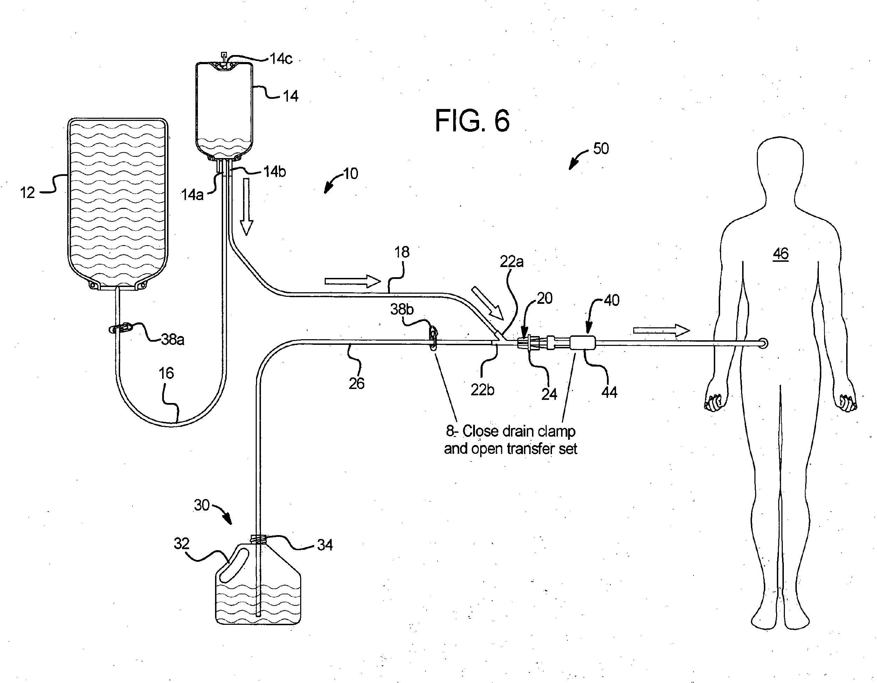

Referring now to Fig. 6 and block 110 of Fig. 3, to accomplish a fill phase, the patient moves the opened clamp 38b on the fill tube 18 to the drain tube 26 and clamps drain tube 26. The patient also twists open transfer set valve 44. Fresh fluid now gravity flows from the smaller supply container 14, through fill tube 18, through junction 20 and the patient's transfer set 40 and into the peritoneal cavity of patient 46. Patient 46 allows smaller supply container 14 to empty the remainder of its dialysate supply into the peritoneal cavity or until patient 46 feels full.

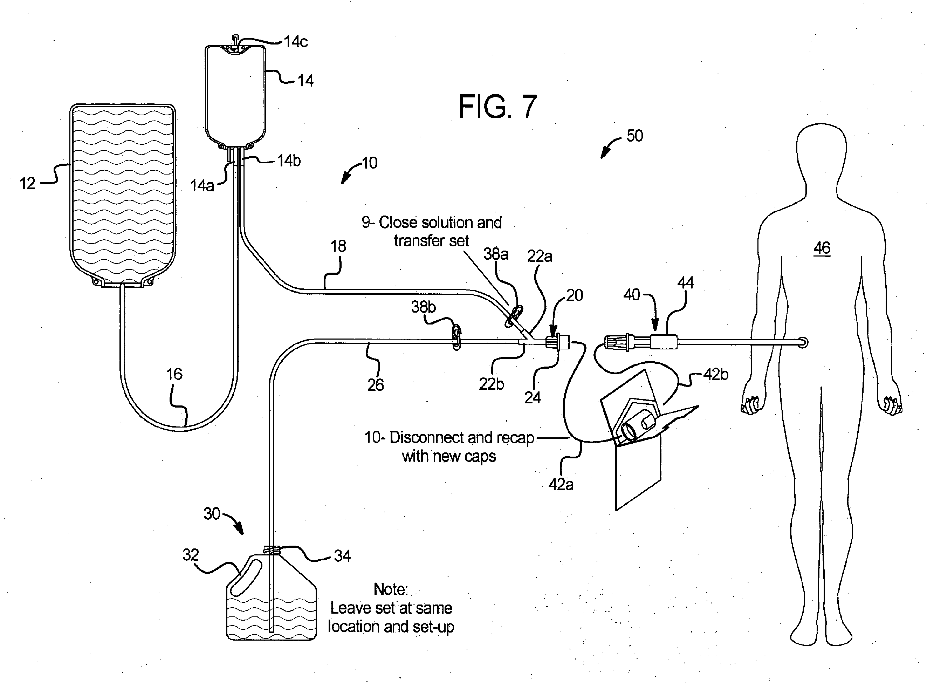

Referring now to Fig. 7 and block 112 of Fig. 3, to accomplish a dwell phase, the patient opens and moves the *previously closed clamp 38a* on supply tube 16 to fill tube 18 and clamps the fill tube *18. Patient 46 closes the valve 44 of transfer set 40* and either closes second clamp 38b to close drain tube 26 or allows clamp 38b to remain closed from the fill phase, such that the fill and drain tubes 18 and 26 are both clamped. Patient 46 then disconnects the patient's transfer set 40 from the patient connector 24 of junction 20 and recaps caps 42a and 42b through a septic process. In one embodiment fresh caps 42a and 42b are used to recap in a proper septic way. In another embodiment, patient connector 24 and transfer set valve 44 are spiked and unspiked and recapped with the same caps 42a and 42b under an ultraviolet ("UV") flash for septic connection and disconnection. In a further embodiment, a sterile connecting device provided by Terumo Corporation, Tokyo, Japan may be used to properly connect and disconnect patient connector 24 and transfer set valve 44 via the same caps 42a and 42b.

In the embodiment illustrated in Fig. 7, smaller supply 14 container is left held elevationally above the larger supply container 12 during dwell, such that smaller container 12 is not refilled at this time. As seen in Figs. 4 to 7, larger supply container 12 is left in place, e.g., on a table, so as to be at approximately a same height as the patient's access, during the entire therapy. Patient 46 can now leave and perform whatever task the patient wishes during the dwell period.

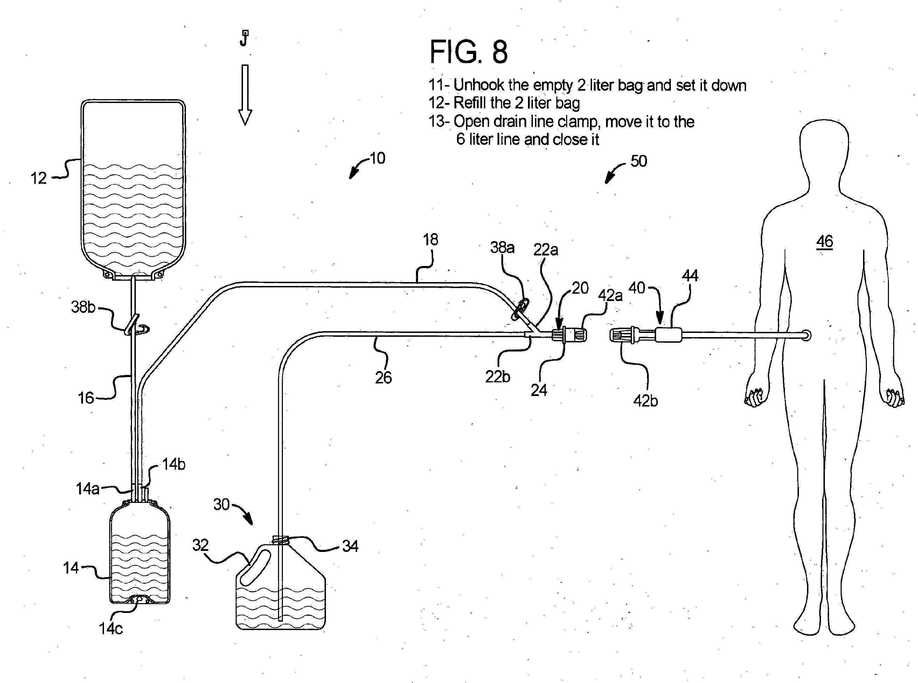

As seen at diamond 114 of Fig. 3, therapy methodology 100 diverges depending upon whether there is another exchange or not. If there is another exchange, as seen at block 116 and Fig. 8, patient 46 moves smaller supply container 14 from the elevated fill position to a relatively low elevational refill position, for example to the floor below larger supply container 12, which has been laid on a table above the lowered smaller supply container 14. Clamp 38a continues to occlude fill tube 18 leftover from the dwell phase. Clamp 38b is moved from the drain line 26 in the dwell phase to supply line 16 but is left open to allow fresh dialysate to gravity flow from the larger supply container 12 to refill the smaller supply container 14. Once supply container is full, patient 46 closes clamp 38b to occlude supply tube 16. All of the above actions in block 116 are shown bordered in broken line labeled 112, indicating that they can be performed during the patient dwell at block 112, saving time once the dwell is completed.

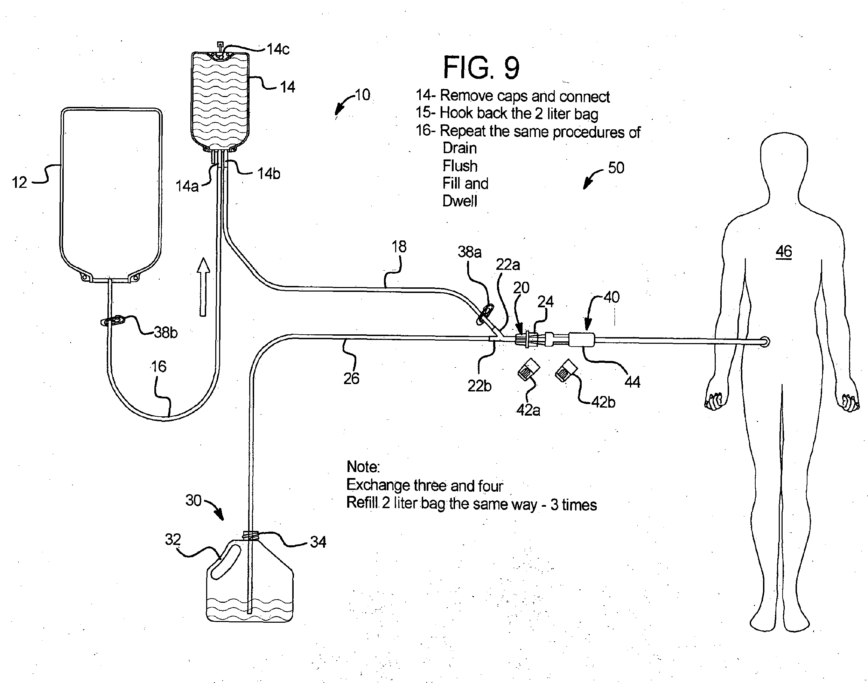

As illustrated in Fig. 9 and block 116 for the next exchange, patient 46 then removes the second set of caps 42a and 42b from patient connector 24 and transfer set 40, respectively, and connects patient transfer set 40 (which is closed) to patient connector 24 of junction 20 of the dialysis set 10. The patient moves the refilled smaller supply container 14 to the elevated position above the larger supply container 12, while ensuring that the supply tube 16 is clamped via clamp 38b to prevent dialysate from flowing back from the smaller supply container 14 to the larger supply container 12. The set is now ready for a second drain, which is initiated when the patient opens his/her transfer set. Drain, flush, fill and dwell are repeated as set forth in blocks 106, 108, 110 and 112, respectively.

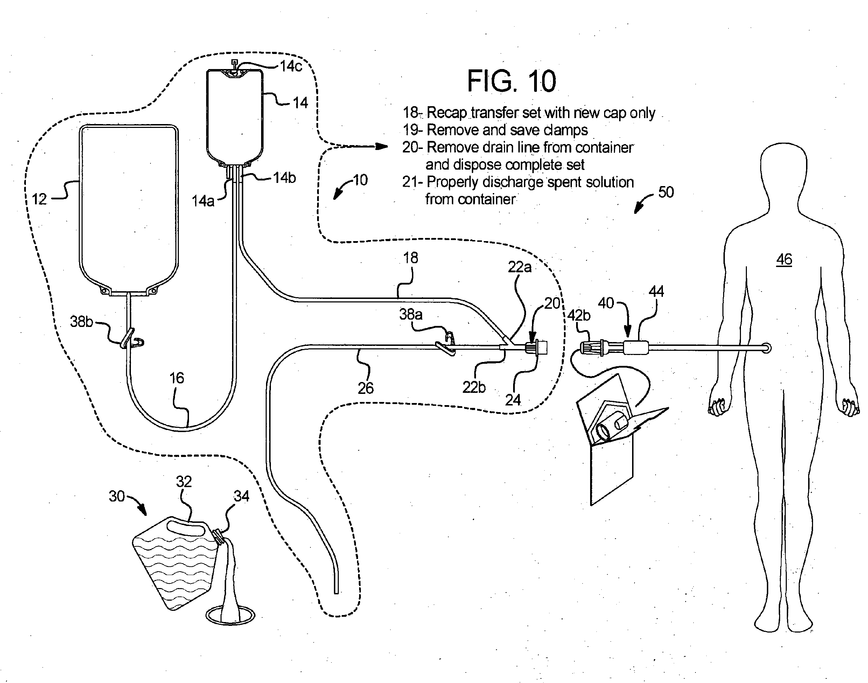

At diamond 114 of Fig. 3, if there is not another exchange, that is when all exchanges, e.g., four, have been completed, patient 46 recaps the transfer set 40 with a new cap 42b, removes and saves the manual clamps 38a and 38b, as seen at block 118 and Fig. 10. The patient removes drain line 26 from drain 30 and empties the reusable container 30, if one is used, and discards the peritoneal dialysis set 10. It is alternatively contemplated to reuse set 10 a number of times such as four times. At oval 120 of Fig. 3, method 100 ends.

### Using A Flow Control Device

In an alternative embodiment, the manual clamping of fill line 18 and drain line 26 is replaced with a flow control device that the patient positions, e.g., turns, to perform the drain, flush, and fill steps. Many embodiments of such a flow control device are set forth in copending U.S. 2009/01403723 ("the '723 Application"), entitled, "Flow Control Device For Peritoneal Dialysis", filed November 29, 2007, assigned to the assignee of the present disclosure.

In particular, Figs. 15 to 19 of the '723 Application show a dial type device, which fit around fill tube 18 and drain tube just prior first port 22a and second port 22b of junction 20. The device eliminates the need for moving, clamping and unclamping individual clamps 38a and 38b at fill tube 18 and drain tube 26. It also eliminates the need for the patient to open and close valve 44 of transfer set 40, which is instead left in the open position unless the patient wants to disconnect from set 10. One of the individual clamps 38a or 38b will still be used to selectively open and close supply tube 16 as discussed above.

It should be understood that various changes and modifications to the presently preferred embodiments described herein will be apparent to those skilled in the art.

## Claims

1. A peritoneal dialysis set (10) comprising:
a larger supply container (12);
a smaller supply container (14) including a supply port (14a) and a fill port (14b), the smaller supply container sized to hold a single patient fill volume of dialysate;
a junction (20) including a patient connector (24) configured to connect to a patient's transfer set (40);
a supply tube (16), wherein the supply port connects the smaller supply container (14) to the larger supply container (12) via the supply tube;
a fill tube (18), wherein the fill port connects the smaller supply container (14) to the junction (20) via the fill tube; and
a drain tube (26) connected to the junction (20).

2. The peritoneal dialysis set (10) of Claim 1, wherein the junction (20) is one of a Y-junction and a T-junction.

3. The peritoneal dialysis set (10) of Claim 1 or 2 which includes first and second manually operated clamps (38a, 38b) for selectively clamping any two of the supply, fill and drain tubes (16, 18, 26).

4. The peritoneal dialysis set (10) of any one of Claims 1 to 3, which includes a flow control device for selectively clamping any two of the supply, fill and drain tubes (16, 18, 26).

5. The peritoneal dialysis set (10) of any one of the preceding claims, wherein at least one of: (i) the larger supply container (12) is a six liter container; and (ii) the smaller supply container (14) is a two liter container.

6. The peritoneal dialysis set (10) of any one of the preceding claims, wherein at least one of: (i) the drain tube (26) is fitted with a removeable plug (28) and (ii) the drain tube (26) is sized to extend to and fit within a drain container (30) when a patient connected to the set (10) is sitting or standing.

7. The peritoneal dialysis set (10) of any one of the preceding claims, wherein the smaller supply container (14) includes an apparatus for hanging the smaller supply container (14).

8. The peritoneal dialysis set (10) of any one of the preceding claims, which includes at least one cap (42a, 42b) to close the junction (20) during at least one patient dwell.

9. The peritoneal dialysis set (10) of any one of the preceding claims, wherein the larger (12) and smaller (14) supply containers are packed full of dialysate.

10. The peritoneal dialysis set (10) of any one of Claims 1 to 8, wherein the larger (12) and smaller (14) supply containers are filled with different types of dialysate.

11. The peritoneal dialysis set (10) of any one of the preceding claims, wherein the larger (12) and smaller (14) supply containers are separated by a frangible plug, which is broken after the smaller supply container (14) is emptied.

12. A peritoneal dialysis system (50) comprising the set (10) of any one of the preceding claims, the system including a drain container (30) configured to receive and secure the drain tube (26).

## Patentansprüche

1. Peritonealdialyseset (10) Folgendes umfassend:
einen größeren Versorgungsbehälter (12);
einen kleineren Versorgungsbehälter (14) mit einem Versorgungsanschluss (14a) und einem Befüllungsanschluss (14b), wobei der kleinere Versorgungsbehälter derart bemessen ist, dass er ein Dialysatfüllvolumen für einen einzelnen Patienten aufnimmt;
ein Verbindungsstück (20) mit einem Patientenanschluss (24), konfiguriert, um an das Transferset (40) eines Patienten angeschlossen zu werden;
einen Versorgungsschlauch (16), wobei der Versorgungsanschluss den kleineren Versorgungsbehälter (14) über den Versorgungsschlauch mit dem größeren Versorgungsbehälter (12) verbindet;
einen Befüllungsschlauch (18), wobei der Befüllungsanschluss den kleineren Versorgungsbehälter (14) über den Befüllungsschlauch mit dem Verbindungsstück (20) verbindet; und
einen Ablaufschlauch (26), in Verbindung mit dem Verbindungsstück (20).

2. Peritonealdialyseset (10) nach Anspruch 1, wobei das Verbindungsstück (20) ein Y-Verbindungsstück oder ein T-Verbindungsstück ist.

3. Peritonealdialyseset (10) nach Anspruch 1 oder 2, eine erste und eine zweite manuell betätigte Klemme (38a, 38b) zum selektiven Klemmen von zwei beliebigen aus Versorgungs-, Befüllungs- und Ablaufschlauch (16, 18, 26) umfassend.

4. Peritonealdialyseset (10) nach einem der Ansprüche 1 bis 3, eine Strömungssteuerungsvorrichtung zum selektiven Klemmen von zwei beliebigen aus Versorgungs-, Befüllungs- und Ablaufschlauch (16, 18, 26) umfassend.

5. Peritonealdialyseset (10) nach einem der vorhergehenden Ansprüche, wobei (i) der größere Versorgungsbehälter (12) ein 6-I-Behälter ist und / oder (ii) der kleinere Versorgungsbehälter (14) ein 2-I-Behälter ist.

6. Peritonealdialyseset (10) nach einem der vorhergehenden Ansprüche, wobei (i) der Ablaufschlauch (26) mit einem entfernbaren Stopfen (28) versehen ist und / oder (ii) der Ablaufschlauch (26) derart bemessen ist, dass er bis zu einem Ablaufbehälter (30) hin gestreckt werden kann und in diesen hineinpasst, wenn ein mit dem Set (10) verbundener Patient sitzt oder steht.

7. Peritonealdialyseset (10) nach einem der vorhergehenden Ansprüche, wobei der kleinere Versorgungsbehälter (14) eine Vorrichtung zum Aufhängen des kleineren Versorgungsbehälters (14) umfasst.

8. Peritonealdialyseset (10) nach einem der vorhergehenden Ansprüche, das wenigstens eine Kappe (42a, 42b) zum Verschließen des Verbindungsstücks (20) während wenigstens einer Patientenverweildauer umfasst.

9. Peritonealdialyseset (10) nach einem der vorhergehenden Ansprüche, wobei der größere (12) und der kleinere (14) Versorgungsbehälter vollständig mit Dialysat gefüllt sind.

10. Peritonealdialyseset (10) nach einem der Ansprüche 1 bis 8, wobei der größere (12) und der kleinere (14) Versorgungsbehälter mit verschiedenen Arten von Dialysat gefüllt sind.

11. Peritonealdialyseset (10) nach einem der vorhergehenden Ansprüche, wobei der größere (12) und der kleinere (14) Versorgungsbehälter durch einen zerbrechbaren Stopfen voneinander getrennt sind, der zerbrochen wird, wenn der kleinere Versorgungsbehälter (14) geleert worden ist.

12. Peritonealdialysesystem (50), umfassend das Set (10) nach einem der vorhergehenden Ansprüche, wobei das System einen Ablaufbehälter (30) umfasst, der konfiguriert ist, den Ablaufschlauch (26) aufzunehmen und zu sichern.

## Revendications

1. Ensemble de dialyse péritonéale (10) comprenant :
un plus grand contenant d'alimentation (12) ;
un plus petit contenant d'alimentation (14) incluant un orifice d'alimentation (14a) et un orifice de remplissage (14b), le plus petit contenant d'alimentation étant dimensionné pour contenir un seul volume de remplissage de dialysat pour patient ;
une jonction (20) incluant un raccord (24) pour patient configuré pour se raccorder à un ensemble de transfert (40) pour patient ;
un tube d'alimentation (16), dans lequel l'orifice d'alimentation raccorde le plus petit contenant d'alimentation (14) au plus grand contenant d'alimentation (12) via le tube d'alimentation ;
un tube de remplissage (18), dans lequel l'orifice de remplissage raccorde le plus petit contenant d'alimentation (14) à la jonction (20) via le tube de remplissage ; et
un tube de drainage (26) raccordé à la jonction (20).

2. Ensemble de dialyse péritonéale (10) selon la revendication 1, dans lequel la jonction (20) est l'une d'une jonction en Y et d'une jonction en T.

3. Ensemble de dialyse péritonéale (10) selon la revendication 1 ou 2, qui inclut des première et seconde brides actionnées manuellement (38a, 38b) permettant de brider sélectivement deux quelconques des tubes d'alimentation, de remplissage et de drainage (16, 18, 26).

4. Ensemble de dialyse péritonéale (10) selon l'une quelconque des revendications 1 à 3, qui inclut un dispositif de régulation d'écoulement permettant de brider sélectivement deux quelconques des tubes d'alimentation, de remplissage et de drainage (16, 18, 26).

5. Ensemble de dialyse péritonéale (10) selon l'une quelconque des revendications précédentes, dans lequel au moins l'un parmi : (i) le plus grand contenant d'alimentation (12) est un contenant de six litres ; et (ii) le plus petit contenant d'alimentation (14) est un contenant de deux litres.

6. Ensemble de dialyse péritonéale (10) selon l'une quelconque des revendications précédentes, dans lequel au moins l'un parmi : (i) le tube de drainage (26) est doté d'un bouchon amovible (28) et (ii) le tube de drainage (26) est dimensionné pour s'étendre vers et s'ajuster au sein d'un contenant de drainage (30) lorsqu'un patient raccordé à l'ensemble (10) est assis ou debout.

7. Ensemble de dialyse péritonéale (10) selon l'une quelconque des revendications précédentes, dans lequel le plus petit contenant d'alimentation (14) inclut un appareil permettant de suspendre le plus petit contenant d'alimentation (14).

8. Ensemble de dialyse péritonéale (10) selon l'une quelconque des revendications précédentes, qui inclut au moins un capuchon (42a, 42b) pour fermer la jonction (20) pendant au moins un temps de pause.

9. Ensemble de dialyse péritonéale (10) selon l'une quelconque des revendications précédentes, dans lequel les plus grand (12) et plus petit (14) contenants d'alimentation sont totalement remplis de dialysat.

10. Ensemble de dialyse péritonéale (10) selon l'une quelconque des revendications 1 à 8, dans lequel les plus grand (12) et plus petit (14) contenants d'alimentation sont remplis de différents types de dialysat.

11. Ensemble de dialyse péritonéale (10) selon l'une quelconque des revendications précédentes, dans lequel les plus grand (12) et plus petit (14) contenants d'alimentation sont séparés par un bouchon cassant, qui est rompu après que le plus petit contenant d'alimentation (14) est vidé.

12. Système de dialyse péritonéale (50) comprenant l'ensemble (10) de l'une quelconque des revendications précédentes, le système incluant un contenant de drainage (30) configuré pour recevoir et assujettir le tube de drainage (26).
